Europäisches Patentamt

**European Patent Office**

Office européen des brevets

(11) Numéro de publication: **0 052 064**
A1

# DEMANDE DE BREVET EUROPEEN

(21) Numéro de dépôt: **81430031.5**

(51) Int. Cl.³: **A 46 B  15/00**

(22) Date de dépôt: **28.09.81**

---

(30) Priorité: **07.10.80  FR 8021548**

(43) Date de publication de la demande: **19.05.82**
**Bulletin 82/20**

(84) Etats contractants désignés: **BE CH DE GB IT LI NL SE**

(71) Demandeur: **Siahou, Joseph, 89, rue d'Endoume,
F-13007 Marseille (FR)**

(72) Inventeur: **Siahou, Joseph, 89, rue d'Endoume,
F-13007 Marseille (FR)**

---

(54) **Brosse à dents ionisante pour utilisation de dentifrices contenant des sels fluorés solubles dans l'eau.**

(57) Réalisation d'une brosse à dents destinée à polariser les principes actifs des dentifrices (par ex. l'ion fluor) par l'incorporation d'un aimant (1) ou d'élastomères magnétiques ou de résines magnétiques ou de deux électrodes (4) et d'une pile (6) ou de deux électrodes (4) d'une pile (6) et d'un aimant (1).

EP  0 052 064  A1

0052064

- I -

## Brosse à dents ionisante

L'objet de l'invention consiste en la réalisation d'une brosse à dents ionisante.

Les brosses à dents,aujourd'hui,ont un rôle
tres important au niveau de l'hygiene dentaire.

L'acte mecanique de brossage bi-quotidien contribue  à l'elimination de la plaque dentaire.

La brosse à dents du  commerce n'a pas d'action
ionisante sur le dentifrice.

Les dentifrices aujourd'hui comprtent du fluor
sous forme de sels.  Il a été demontré le role
du fluor dans la prevention contre la carie,
ainsi que son rôle au niveau de la desensibilisation des collets denudés des  dents.

L'objet de cette brosse ionisante est de demultiplier l'action de l'ion F et des autre s
principes actifs et de les entrainer dans les
endroits inaccessibles au brossage habituel
(poches,culs de sacs,zones intermediares).

- 2 -

Le mouvement de brossage etant dynamique, mouvement vertical:
brossage de haut en bas pour l'arcade superieure
brossage de bas en haut pour l'arcade superieure,
la création d'un champ pour polariser l'ion F va entrainer un changement des trajectoires de cet ion.

L'intensité d'action d'un element suivant son etat (tres grande inertie chimique et tres grande activité physiologique ou vice versa) rapelle les differences d'activités des corps dans les reactions chimiques suivant leur degré d'activation. Elles sont comparables à l'activité des corps naissants.

L'objet de cette invention consiste en la realisation d'une brosse à dents dont les composants peuvent etre differents ou qui peut comporter en plus des additifs qui favorisent l'action du Fluor ou d'autres principes actifs.

Brosse à dents où est incorporé un aimant: il a été demonté, depuis peu d'années; qu'une solution ionisée en presence d'un champ magnetique ou electro magnetique entraine un effet certain sur les ions de cette solution.

Cet aimant, incorporé au niveau de la tête de la brosse à dents, peut-être par exemple:

- 3 -

- un aimant ferrite
- un aimant Ticonal ou Ticonium
- un aimant supra conducteur
- un aimant permanent conducteur
- un aimant aux terres rares

ceci ne constituant pas une liste limitative.

Brosses à dents constituées par des elastomeres magnetiques.
Les resines constituant la tete de la brosse à dents peuvent être remplacées par des elastomeres magnetiques.

Brosses à dents constituées par des resines magnetiques:
Les resines elles mêmes peuvent être magnetiques et avoir le même effet que l'aimant.

Brosses à dents comportant une pile et deux electrodes/:
Le passage d'un faible courant permet de polariser l'ion et de le rendre encore plus actif.

Brosses à dents comportant une pile, deux electrodes et un aimant:
C'est une brosse comportant un champ electro-magnetique.

Revendications de brevet

I; Dispositif d'ionisation de la brosse à dents destiné à la polarisation des principes actifs(ions FLUOR) du dentifrice à sels ionisés se caracterisant par le fait que la tête de la brosse (I) comporte un aimant.

2. Dispositif suivant la revendication I se caracterisant par le fait que la tête de la brosse à dents ( I) est constituée par une resine magnetique.

3. Dispositif suivant la revendication I se caracterisant par le fait que la tete d. la brosse à dents (I) est constituée par un elastomere magnetique.

4. Dispositif suivant la revendication I se caracterisant par le fait que la tete de la brosse à dents(2) comporte deux electrodes (4) reliées par un fil electrique(5) à une pile (6)placée au bout du manche ainsi qu'un aimant dans la tete (I).

1/1

0052064

FIG1

FIG 2

FIG 3

0052064

Numéro de la demande

**Office européen des brevets**

**RAPPORT DE RECHERCHE EUROPEENNE**

EP 81 43 0031

| | **DOCUMENTS CONSIDERES COMME PERTINENTS** | | | **CLASSEMENT DE LA DEMANDE (Int. Cl. 3)** |
|---|---|---|---|---|
| Catégorie | Citation du document avec indication, en cas de besoin, des parties pertinentes | | Revendication concernée | |
| | FR - A - 2 155 338 (NARCOHM SEISA-KUSHO) <br><br> * en entier * | | <br><br> 1,4 | A 46 B 15/00 |
| | --- | | | |
| | GB - A - 956 126 (LION BRUSH) <br><br> * en entier * | | <br><br> 1,4 | |
| | --- | | | |
| | FR - A - 2 218 860 (RAULO) <br><br> * en entier * | | <br><br> 1,4 | **DOMAINES TECHNIQUES RECHERCHES (Int. Cl. 3)** |
| | --- | | | |
| | FR - A - 1 558 852 (SIMOR) <br><br> * en entier * | | <br><br> 1,4 | A 46 B |
| | --- | | | |
| | US - A - 1 831 393 (PIERCE Jr.) <br><br> * en entier * | | <br><br> 1-4 | |
| | --- | | | |
| | US - A - 4 017 386 (BARRY) <br><br> * en entier * | | <br><br> 1-4 | |
| | --- | | | |
| | US - A - 2 426 315 (MARICK) <br><br> * en entier * | | <br><br> 1-3 | **CATEGORIE DES DOCUMENTS CITES** |
| | --- | | | X: particulièrement pertinent à lui seul |
| | DE - A - 2 646 265 (RICHTER) <br><br> * en entier * | | <br><br> 1 | Y: particulièrement pertinent en combinaison avec un autre document de la même catégorie <br> A: arrière-plan technologique <br> O: divulgation non-écrite <br> P: document intercalaire <br> T: théorie ou principe à la base de l'invention <br> E: document de brevet antérieur, mais publié à la date de dépôt ou après cette date <br> D: cité dans la demande <br> L: cité pour d'autres raisons |
| | -------- | | | |
| | | | | &: membre de la même famille, document correspondant |

Le présent rapport de recherche a été établi pour toutes les revendications

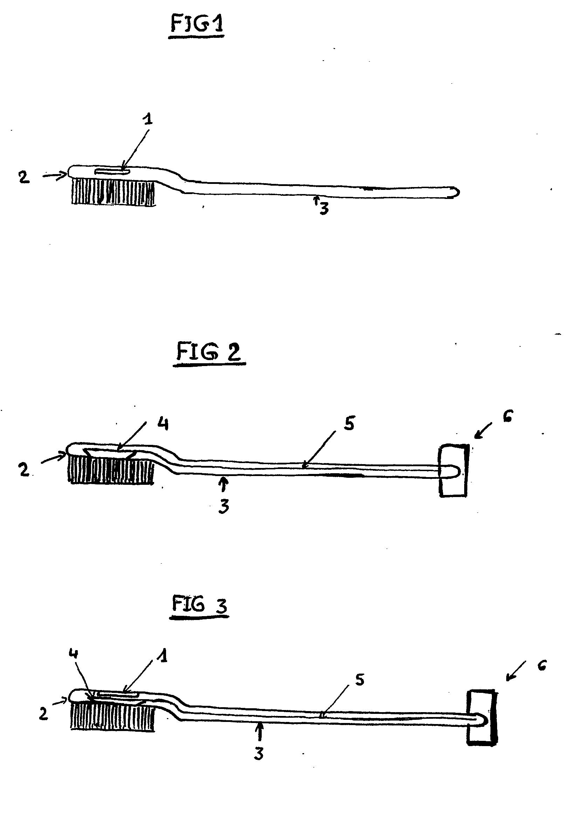

| Lieu de la recherche | Date d'achèvement de la recherche | Examinateur |
|---|---|---|
| La Haye | 08.01.1982 | BOURSEAU |

OEB Form 1503.1  06.78